# EUROPEAN PATENT APPLICATION

(11) **EP 4 799 652 A1**
(43) Date of publication of application: **02.09.2026**
(21) Application number: 25160828.7
(22) Date of filing: 28.02.2025
(51) Int. Cl.: A61L 2/08, A61B 1/07, A61L 2/10, A61B 1/06

(54) **A LIGHT DISINFECTION DEVICE WITH A PROTECTIVE CAP**

(71) Applicant: UV Clinical A/S, 2900 Hellerup (DK)
(72) Inventor: Bak, Jimmy, 2670 Greve (DK)
(74) Representative: Høiberg P/S

(57) **Abstract**

The present disclosure relates to a liquid lightguide assembly which is a kit of parts comprising a light disinfection device and a protective cap. The present disclosure further relates to a method for preventing the formation of air pockets in light disinfection devices. The disclosure further relates to a protective cap for safe storage of a liquid-filled elongated tubular body, and to a method for prevent the formation of air pockets in a light disinfection device.

## Description

The present disclosure relates to a lightguide assembly which is a kit of parts comprising a light disinfection device and a protective cap. The present disclosure further relates to a method for preventing the formation of air pockets in lightguide disinfection devices.

### Background

Catheters are widely used in clinical procedures worldwide. Specifically, certain catheters, such as central venous catheters, may need to be inserted in patients for an extended period. Using such a device for a prolonged period is associated with risks, as a catheter may be colonized with hospital-acquired organisms.

Specifically, central venous catheters (CVCs) are widely used in medical settings for the administration of medications, fluids, and nutrients, as well as for monitoring hemodynamic parameters. However, CVCs are also associated with a significant risk of infection due to microbial colonization on both the internal and external surfaces of the catheter. Bacteria and fungi can adhere to the catheter surface, forming a biofilm that provides a protective environment for microbial growth. This biofilm can serve as a persistent source of infection, with microorganisms detaching and entering the bloodstream, leading to catheter-related bloodstream infections (CRBSls). These infections can cause systemic inflammatory responses, sepsis, and, in severe cases, multi-organ failure. The risk is heightened by the prolonged presence of the catheter in the bloodstream and the difficulty in eradicating established biofilms using conventional antimicrobial treatments. Given the direct access to the central circulation, colonization of CVCs poses a serious health risk, requiring effective strategies for preventing microbial growth and maintaining catheter sterility.

To tackle the above problem, UV lightguides have been developed to disinfect the catheters. Elongated light delivery systems emitting UV light from LEDs such as small diameter optical glass fibers and liquid light-guides to disinfect the lumen of medical devices, such as catheters and endotracheal tubes are known. For instance, see WO 2013/023666 by the same applicant, which is incorporated herein by reference in its entirety. These elongated liquid light-guides typically emit light continuously and radially along the length of the guide and homogeneously around the circumference of the light guide. The liquid lightguides used for disinfection of the inner lumen of medical tubes and catheters are filled with a high concentration ionic solution, for instance sodium chloride or magnesium chloride or calcium chloride. These solutions are transparent at UV- VIS wavelengths and have an enhanced refractive index compared to the thin fluoropolymer wall encapsulating the ionic solution.

However, there is a risk that liquid light-guides may leak parts of the ionic solution from the guide. Leakage of the ionic solution, from the liquid light-guide often results in contamination of the optical surfaces of the light source. This reduces the light transmittance and hence disinfection efficacy of the light guide. More importantly, a leakage from the liquid light-guide creates an air space or air bubbles in the light guide, which often results in an abrupt loss of light by scattering and consequently a decrease in the transmittance through the entire length of the light guide.

Moreover, the liquid lightguides which are part of the light disinfection devices comprise very thin-walled tubes made by hard polymer materials, such as fluoropolymers. It is a frequent problem that such tubes kink and are easily damaged during transportation or storage of such devices.

Hence, there is a need for a liquid lightguide and a procedure for filling the lightguide with a transparent ionic solution which can both prevent the formation of air bubbles and at the same time can prevent that the light guide is damaged during storage and transportation.

### Summary

One purpose of the present disclosure is to reduce the formation of air bubbles in a liquid-carrying lumen of a light disinfection device, as well as minimize damage, such as kinking, of such light disinfection devices. Specifically, the present disclosure relates to a lightguide assembly, which is a kit of parts comprising a light disinfection device comprising an elongated tubular body and a connector piece connectable to a light source. In the following sections the term lightguide assembly refers to the kit of parts comprising the light disinfection device comprising an elongated tubular body and the connector piece connectable to a light source

The elongated tubular body may comprise a transparent tube wall having a refractive index n₂, and a proximal end closed by a first closure and connectable to the connector piece. The first closure may be made by a watertight and transparent material. The elongated tubular body may further comprise a distal end closed by a second closure, wherein the tube wall, the first closure and the second closure define a closed inner lumen. A light guiding liquid is then typically retained within the closed inner lumen, the light guiding liquid preferably having a refractive index n₁, wherein n₂/n₁ < 1 so that the elongated tubular body is configured for guiding light from the light source inside the liquid filled inner lumen along a longitudinal direction of the elongated tubular body, wherein a part of the light is emitted through the tube wall, such that the light disinfection device is configured for emitting light from, typical radially from, and along the length of the elongated tubular body.

The lightguide assembly may further comprise a protective cap for safe storage of the elongated tubular body, the protective cap comprising an elongated cover for encapsulating the elongated tubular body. The elongated cover may comprise a closed distal end, a proximal open end for receiving the distal end of the elongated tubular body, and a proximal connector in connection with the proximal open end configured for engagement with the connector piece of the light disinfection device to form a closed outer lumen encapsulating the elongated tubular body. This closed outer lumen is advantageously configured for retaining a fluid, such as a liquid or an ionic solution. In the following this fluid will be referred to as "a protective fluid", because it is provided to encapsulate and "protect" the elongated tubular body during storage and/or transport. Preferably the protective fluid is similar to the light guiding liquid inside the elongated tubular body.

One advantage of the protective cap is that it enables safe and secure storage of the light disinfection device. By encapsulating the elongated tubular body, it is possible to prevent atmospheric gases from penetrating the wall of the elongated tubular body, thereby reducing the formation of air bubbles in the closed inner lumen. The presence of air bubbles creates challenges, as air bubbles could attenuate light propagation and consequently affect the emission of light along the length of the elongated tubular body. The protective cap provides an environment free of air bubbles which results in a stable light transmission and consequently an optimal performance of the light disinfection device.

In addition, a further advantage of the protective cap is its ability to safeguard the structural integrity of the elongated tubular body during storage, transportation, and handling. The cap prevents mechanical damage, such as kinking or deformation of the tubular body, which could compromise the device's ability to guide light effectively. The use of a robust material for the cap, a material comprising potentially UV-resistant and impact-resistant polymers, enhances its protective properties, making the cap durable and reliable under a variety of conditions. Moreover, the protective cap could be made of a non-transparent polymer which could be used to prevent e.g. UV light from leaking to the environment if the light source is unintentionally switched on before the protective cap is removed.

In one embodiment, the protective cap may include additional features that further enhance its utility. For instance, the proximal connector may employ a threaded or bayonet-style locking mechanism to securely attach the cap to the connector piece of the light disinfection device. Alternatively, a snap-fit mechanism could allow for quick and effortless attachment and removal of the cap. Another option is a Luer male-female connector. The design may also incorporate sealing elements, such as O-rings or elastomeric gaskets, to ensure a fluid-tight seal between the protective cap and the light disinfection device, preventing the ingress of contaminants or the egress of protective fluid.

Moreover, the lightguide assembly may comprise a protective sleeve configured to encapsulate the light disinfection device. The protective sleeve may be configured to be selectively elongated or contracted along the axis of the light disinfection device. An advantage of incorporating a protective sleeve is that the protective sleeve can be pulled over the lightguide to protect against contamination when the lightguide is inserted into the catheter.

Furthermore, the present disclosure relates to a method for preventing the formation of air pockets or air bubbles in the light disinfection device. The method comprises the steps of obtaining a light disinfection device, the light disinfection device comprising an elongated tubular body, said elongated tubular body comprising a liquid, encapsulating the elongated tubular body within a protective cap, and filling the interface between the protective cap and the elongated tubular body with a protective liquid, thereby preventing any liquid from escaping the interface between the elongated tubular body

and the protective cap, and preventing air from entering the interface between the elongated tubular body and the protective cap.

### Description of Drawings

Various embodiments are described hereinafter with reference to the drawings. The drawings are examples of embodiments and are intended to illustrate some of the features of the presently disclosed light disinfection device with a protective cap and are not limiting to the presently disclosed system and method.
Fig. 1 shows a schematic of a light disinfection device according to one embodiment of the present disclosure.
Fig. 2 shows a schematic of an example of a protective cap.
Fig. 3 shows a side-view schematic of an example of a protective cap.
Fig. 4 shows an isometric view of an embodiment of an example of a protective cap mounted on the light disinfection device.
Fig. 5 shows a side-view of an example of a protective cap engaged on an embodiment of the light disinfection device.
Fig. 6 shows a side-view of an example of a protective sleeve engaged on an embodiment of the light disinfection device.
Fig. 7 shows an isometric view of an example of an elongated protective sleeve.
Fig. 8 shows an isometric view of an example of a contracted protective sleeve.

### Reference signs used in the drawings

100: light disinfection device
101: female connector part of the light disinfection device
102: elongated tubular body
103: optical window
104: O-ring
105:tube seal
106: Luer connector
107: connector piece
108: proximal end of the elongated tubular body
109: distal end of the elongated tubular body
110: transparent tube wall
111: light guiding liquid
200: elongated cover of the protective cap
201: closed distal end of the elongated cover
202: proximal open end of the elongated covert
203: proximal connector
204: protective cap
300: side-view of a protective cap
400: protective cap
401: connector piece
500: protective cap
501: Luer connector
502: connector piece of the light disinfection device
600: protective sleeve
700: isometric view of an elongated protective sleeve
701: light disinfection device
800: contracted protective sleeve

### Detailed description

A purpose of the present disclosure is to enhance the quality of light disinfection devices, especially during storage of such devices. Specifically, a common problem of such devices is the introduction of air into the elongated tubular body, which results in decreased efficiency of the light disinfection properties. Additionally, as such tubular bodies are made of hard fluoropolymer materials, another common problem is that they deform easily if bended or stretched too much, which significantly reduces the light propagation properties of the light disinfection device.

Therefore, to address such challenges, the present disclosure relates to a lightguide assembly comprising a light disinfection device and a protective cap. The light disinfection device may comprise an elongated tubular body and a connector piece connectable to a light source. The elongated tubular body may comprise a transparent tube wall having a refractive index n₂, a proximal end closed by a first closure and connectable to the connector piece, a distal end closed by a second closure, wherein the tube wall, the first closure and the second closure define a closed inner lumen, and a light guiding liquid retained within the closed inner lumen, the light guiding liquid having a refractive index n₁, wherein n₂/n₁ < 1 so that the elongated tubular body is configured for guiding light from the light source inside the liquid filled inner lumen along a longitudinal direction of the elongated tubular body, wherein a part of the light is emitted through the tube wall, such that the light disinfection device is configured for emitting light radially from and along the length of the elongated tubular body. In an embodiment, the proximal end may be closed by a watertight and transparent first closure. It is important that n₂/n₁ < 1 to assist light guiding by internal reflections inside the tubular body, enabling the light to travel along the length of the tube with a user determined leakage of light though the tube wall. Internal reflection of light is promoted for light rays incident at angles greater than the critical angle. The degree of propagation along the light guide and transmittance through the tube wall can be controlled by the concentration of the specific transparent ionic solution. Such a process can result in low attenuation and efficient transmission of light through the light disinfection device.

The protective cap can be used for safe storage of the elongated tubular body. The protective cap may comprise an elongated cover for encapsulating the elongated tubular body, wherein the elongated cover may comprise a closed distal end, a proximal open end for receiving the distal end of the elongated tubular body, and a proximal connector in connection with the proximal open end configured for engagement with the connector piece of the light disinfection device to form a closed outer lumen encapsulating the elongated tubular body for retaining a protective fluid. A function of the protective fluid is to prevent atmospheric gasses to penetrate the thin fluoropolymer wall of the light disinfection device and create air bubbles in the inner lumen. In an embodiment, the proximal connector is configured for engagement with the distal end of the connector piece of the light disinfection device. Specific engagement mechanisms may be used, such as having a threaded connection or a snap-fit mechanism. Depending on the type of application and the characteristics of the light disinfection device, a certain type of engagement mechanism may be chosen.

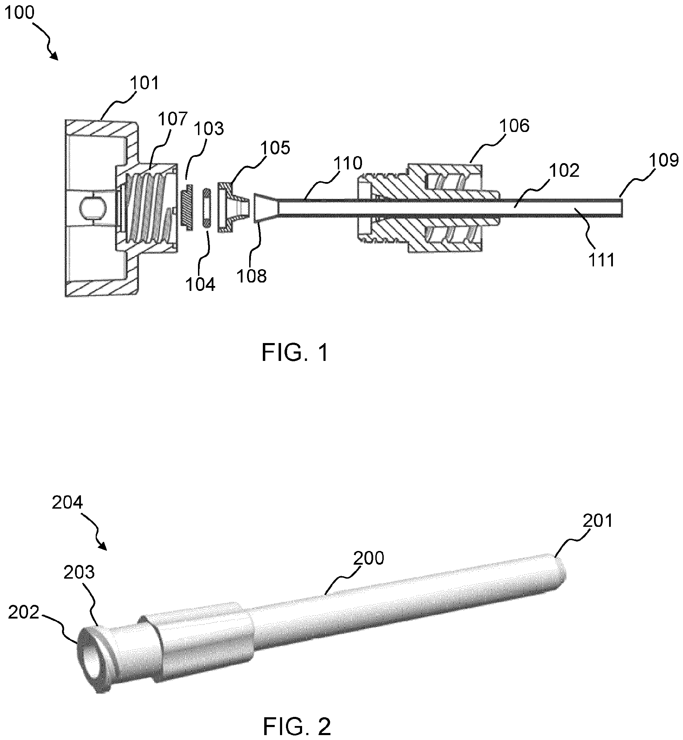

An example of the disclosed light disinfection device is shown in Fig. 1. The light disinfection device 100 may comprise various components. The female connector part 101 is connected to a light source such that light emitted from the light source can be transmitted into the elongated tubular body 102 through the optical window 103. The light then propagates towards the transparent tube wall 110 of the elongated tubular body 102. The female connector part may comprise a connector piece 107and an optical window 103 which allows light to be transmitted the elongated tubular body. The light disinfection device may further comprise an O-ring 104 arranged between the connector piece and the elongated tubular body. The O-ring can be in contact with the optical window to provide a watertight closure. A tube seal 105 may be used in order to secure the elongated tubular body to the connector. Various connector mechanisms may be used, such as threaded or snap-fit connections. The light disinfection device may also comprise a Luer connector 106 configured for engagement with the connector piece of the light disinfection device. The elongated tubular body comprises a proximal end 108 and a distal end 109. As a result, the light guiding liquid 111 can be contained within the closed inner lumen that is formed from the transparent tube wall, the proximal end and the distal end.

Figure 2 shows an example of a protective cap 204. In this embodiment, the protective cap comprises an elongated cover 200 for encapsulating the elongated tubular body of the light disinfection device. The elongated cover comprises a closed distal end 201, a proximal open end 202 for receiving the distal end of the elongated tubular body, and a proximal connector 203. In this embodiment, the proximal connect is a Luer thread.

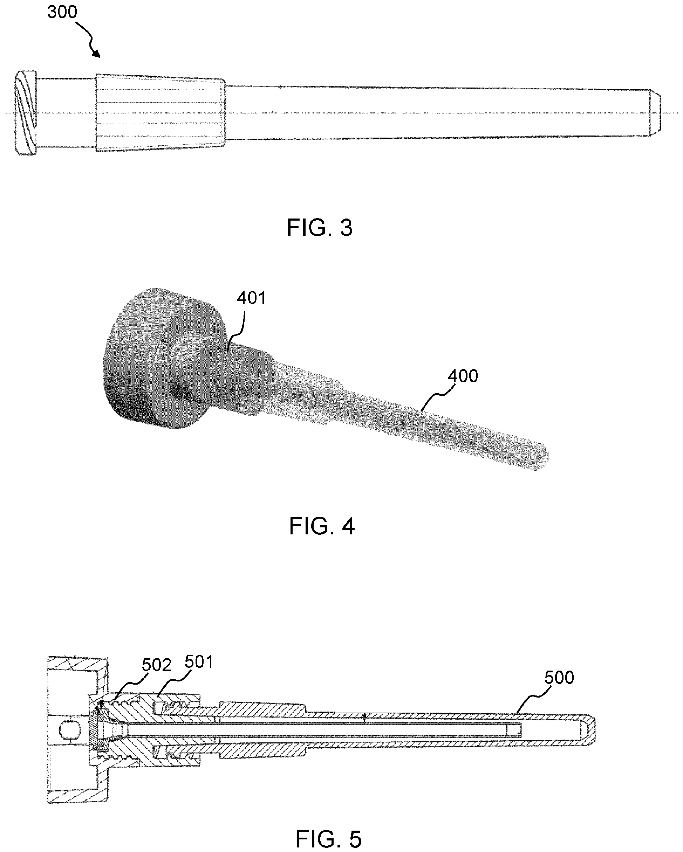

Figure 3 shows a side-view schematic of a protective cap 300, and figure 4 shows a schematic where a protective cap 400 is engaged on the connector piece 401 of a light disinfection device.

Fig. 5 shows an example of a protective cap 500 being engaged on a light disinfection device via engagement using a Luer connector 501. The protective cap can be engaged on the connector piece 502 of the light disinfection device, effectively securing the protective cap. Such an assembly has significant advantages, as it is known that light disinfection devices are partly permeable to atmospheric gases, resulting in the creation of air bubbles in the ionic solution enclosed in a light disinfection device. Air bubbles scatter and attenuate the transmitted light through the light disinfection device, effectively decreasing its efficiency to propagate and distribute light. Additionally, there is a risk that the state of the art light disinfection devices are damaged during storage as the elongated tubes are deformed, stretched and kinked, resulting in a damaged device. Therefore, the present assembly utilizing the protective cap has significant advantages, as it can protect the light disinfection device from kinking, and it can also prevent atmospheric gases from entering the lumen of the light disinfection device. As a result, the protective cap can expand the shelf lifetime of a light disinfection device, and minimize the risk that a light disinfection device is damaged during storage, transport or handling.

In an embodiment, the lightguide assembly can be configured such that the protective fluid is a protective liquid. In an embodiment, the protective fluid may be an ionic solution comprising solutes which are transparent, outgassed for atmospheric molecules and the ionic solution can match the concentration of the ionic solution contained in the elongated tubular body. As a result, no water molecules can be exchanged to the environment and at the same time only atmospheric gases in very small concentrations are present on each side of the fluoropolymer wall.

Moreover, the protective cap can be configured to seal the elongated body, thereby preventing any liquid from escaping the closed outer lumen encapsulating the elongated tubular body and preventing air from entering the closed inner lumen and the closed outer lumen. By preventing air ingress, the present configuration reduces the likelihood of air bubbles forming within the light-guiding liquid, which could disrupt the optical properties and impair light transmission. Sealing the device also ensures that the protective fluid remains uncontaminated, preserving its ability to shield the tubular body from environmental factors.

In an embodiment, the lightguide assembly can further comprise a protective sleeve configured to encapsulate the light disinfection device. The protective sleeve may provide an additional layer of physical protection, shielding the elongated tubular body and its components from mechanical stresses, such as bending or impacts. Using a protective sleeve can be particularly beneficial during transportation or when a light disinfection device is stored alongside other equipment.

In an embodiment, the protective sleeve can be configured to be selectively elongated or contracted along the axis of the light disinfection device. Such an adjustable feature enables the protective sleeve to conform to different device configurations, ensuring compatibility and flexibility in various usage scenarios. For example, the protective sleeve can be pulled over the light disinfection device to protect against contamination when the device is inserted into the catheter. Additionally, the protective sleeve may reduce UVC exposure to the surroundings.

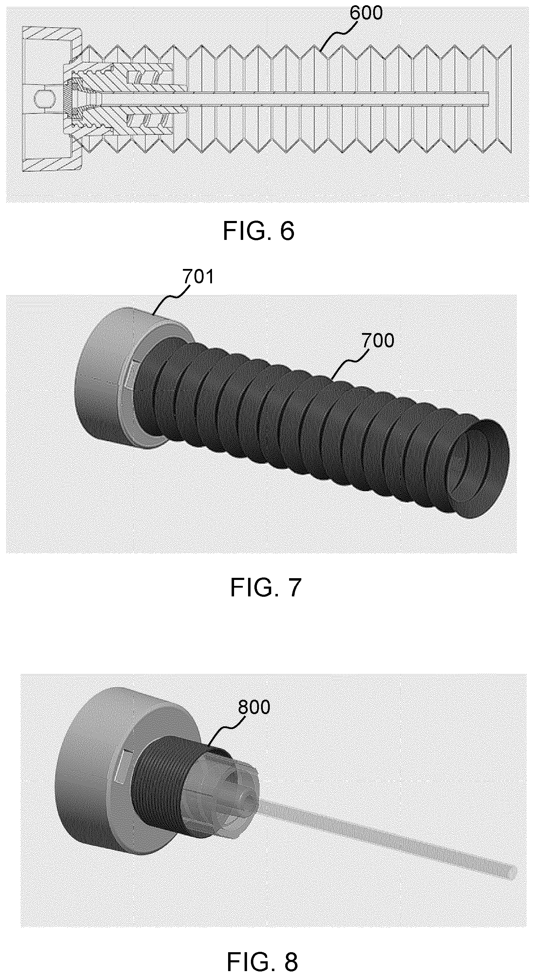

Figure 6 shows an example of a protective sleeve 600 engaged on a light disinfection device. Figure 7 shows an isometric view of an elongated protective sleeve 700 being engaged on a light disinfection device 701, while figure 8 shows an example of a contracted protective sleeve 800.

The protective sleeve can be made by different materials, such as plastic or fabric, and it may have customizable shape and size depending on the type of the light disinfection device.

Furthermore, the lightguide assembly can be configured such that the protective fluid is the same as the light guiding liquid. In an embodiment, the protective fluid and/or the light guiding liquid may be an ionic solution, such as a saline solution. For example, certain compounds that can be used are: sodium chloride, magnesium chloride and calcium chloride Ionic solutions are particularly advantageous because of their high transparency and ability to transmit UV light with minimal scattering. Additionally, saline solutions are non-toxic, readily available, and easy to handle, making them ideal for medical applications.

The lightguide assembly can be configured, such that the protective fluid and/or the light guiding liquid is a saline solution, wherein a salt concentration of the saline solution is at least 10 g/100 ml, or at least 20 g/100 ml, or at least 30 g/100 ml. Even higher salt concentrations may further increase the refractive index of the liquid, improving light-guiding efficiency and enhancing the overall optical performance of the light disinfection device.

In an embodiment, the protective cap is made of a polymer. Polymers offer several advantages, including durability, lightweight properties, and resistance to environmental stressors. For example, the protective cap may comprise biocompatible materials or materials selected from the group of: polypropylene, polystyrene, nylon, polycarbonate and methacrylate. Using such materials ensures that the protective cap can withstand mechanical impacts, maintain structural integrity, and provide long-term reliability during storage and transport. The choice of the material of the protective cap can depend on specific requirements, such as resistance to UV radiation, chemical stability, or mechanical strength. For instance, polycarbonate may be preferred for its high impact resistance, while methacrylate may be used for its optical clarity and ease of moulding.

Moreover, the lightguide assembly can be configured, such that the protective cap is made of a transparent material. Transparency allows for visual inspection of the encapsulated elongated tubular body without removing the protective cap, providing convenience and ensuring the device's readiness for use. A transparent cap also facilitates the detection of any potential issues, such as contamination, damage, or leakage, enabling quick corrective actions before deployment of the device.

Furthermore, the lightguide assembly can be configured, such that the connector piece of the light disinfection device is a Luer lock-type connector. A Luer lock may provide a secure and reliable connection between the light disinfection device and the protective cap, and between the light disinfection device and a catheter, preventing unintentional disconnection during operation. This type of connector has certain advantages such as ease of use, standardization, and ability to form a tight seal, ensuring optimal light transfer into the liquid-filled inner lumen of the tubular body.

In an embodiment, the lightguide assembly can be configured such that the proximal connector of the protective cap is a Luer lock-type connector. Such a Luer lock-type connector can ensure compatibility with the connector piece of the light disinfection device, facilitating a secure and quick engagement between the cap and the device.

The lightguide assembly can be configured such that the connector piece is configured for connecting the light disinfection device to a medical device or to a medical tube, such as a catheter. Such a feature makes the light disinfection device versatile and applicable in various clinical settings, where precise light delivery is necessary. For example, the connector piece may allow the device to interface seamlessly with catheters used for disinfection of their internal lumens.

In an embodiment, the lightguide assembly can be configured such that the connector piece is configured for secure connection with a venous catheter having a lumen. This configuration ensures that the light disinfection device can be used effectively in vascular access applications, where disinfection of internal surfaces is critical. The secure connection minimizes the risk of disconnection during use, maintaining consistent and reliable light delivery.

In addition, the lightguide assembly can be configured such that the connector piece comprises a threaded or snap-fit locking mechanism for securing the light disinfection device to the light source. A threaded mechanism provides an adjustable and tight connection, while a snap-fit design allows for quick attachment and detachment. Both configurations ensure stability during operation, preventing misalignment or unintended disconnection. Depending on the type of application, a different type of locking mechanism may be chosen.

The lightguide assembly can be configured such that the connector piece of the light disinfection device comprises a threaded or snap-fit locking mechanism for engagement with the proximal connector of the protective cap. This feature enables the protective cap to be securely attached to the device, ensuring that the elongated tubular body remains encapsulated and protected during storage or transport.

In an embodiment, the lightguide assembly can be configured such that the connector piece of the light disinfection device is configured for sliding along the elongated tubular body, between the first closure and the second closure. Such a sliding functionality provides flexibility in positioning the connector piece, allowing for optimal alignment with the light source or other interfacing components. The ability to adjust the position of the connector piece enhances the versatility and usability of the device.

Moreover, the lightguide assembly can be configured, such that the connector piece of the light disinfection device comprises a female connector part for engaging with a male connector part of the light source. This configuration ensures a secure and straightforward connection between the light source and the device, facilitating efficient light transfer into the liquid-filled inner lumen. The connector part can for instance be a bayonet coupling part.

In an embodiment, the lightguide assembly can be configured, such that the proximal connector of the protective cap comprises a male connector part for engaging with a female connector part of the connector piece of the light disinfection device. This design enables the protective cap to form a secure and sealed connection with the light disinfection device, ensuring that the encapsulated tubular body remains protected from external contaminants and mechanical damage.

The lightguide assembly can be configured such that the connector piece of the light disinfection device and/or the proximal connector of the protective cap is made of a UV-resistant material. Such a feature can ensure durability and prevents degradation of the components when exposed to UV or UVC light during operation or storage. Using UV-resistant materials enhances the longevity of the device, particularly in applications where UV light is the primary disinfecting agent. In addition, using UV-resistant and non-transparent materials ensures that the light does not propagate to the environment.

Moreover, the lightguide assembly can be configured such that the connector piece of the light disinfection device comprises an adjustable locking collar for enabling varying degrees of tightness when securing the proximal end of the elongated tubular body. Using such a collar allows a user to adjust the connection based on specific operation needs, ensuring a secure attachment while accommodating potential variations in component dimensions.

In addition, the lightguide assembly can be configured such that the connector piece of the light disinfection device comprises multiple connection interfaces, such as bayonet and threaded connections for accommodating different types of light sources. This feature ensures compatibility with a wide range of light sources, providing flexibility in adapting the device for different applications. Using multiple connection interfaces enables the device to meet various user preferences and operational requirements.

In an embodiment, the lightguide assembly can be configured such that the connector piece of the light disinfection device comprises a rotatable joint, enabling the light disinfection device to rotate 360º while maintaining a secure connection to the light source. Having such a rotational capability enhances usability by allowing the light disinfection device to be oriented freely without disconnecting the device from the light source. This feature is particularly useful in confined or complex operational settings, such as clinical procedures involving intricate lumens.

The lightguide assembly can be configured such that the connector piece comprises a controllable shutter configured to block transmission of light to first closure of the light disinfection device. This feature provides additional control over light propagation, allowing the user to prevent light emission when the device is not in active use. The controllable shutter enhances safety and prevents unintended exposure to UV light, protecting users and the surrounding environment.

Furthermore, the lightguide assembly can be configured such that the light disinfection device comprising a light coupling element. The light coupling element may comprise an optical window, a distal end configured for engagement with the first closure, and a proximal end configured for engagement with the light source, such that the light from the light source is guided into the light disinfection device. Such an arrangement can ensure efficient light transfer into the liquid-filled inner lumen, optimizing the device's disinfection performance.

The lightguide assembly can be configured such that the distal end of the light coupling element comprises a seal. Such a seal can ensure a fluid-tight connection with the first closure of the device. The seal can prevent the ingress of contaminants, and it can maintain the integrity of the liquid-filled inner lumen. The inclusion of a seal also enhances the reliability of the light coupling element in various environmental conditions.

In addition, the lightguide assembly can be configured such that the light coupling element comprises an O-ring positioned between the optical window and the distal end of the light guiding element. The O-ring provides an additional sealing layer, preventing leaks and ensuring that the light coupling element maintains a secure and reliable connection with the elongated tubular body. The optical window may comprise a UVC transparent polymer material, such as cyclic olefin copolymer which in some formulations is UVC transparent, for instance TOPAS. The optical window and the O-ring seal the elongated tubular body and prevent the ionic solution from leaking to the environment.

The lightguide assembly can be configured such that the connector piece is configured to secure the light coupling element between the light source and the elongated tubular body. This configuration ensures that the light coupling element remains properly aligned during operation, facilitating efficient light transfer and minimizing losses.

In an embodiment, the lightguide assembly can be configured such that the material of the elongated tubular body is selected from the group of: Teflon, Fluorinated ethylene propylene (FEP), polytetrafluoroethylene (PTFE), amorphous fluoropolymers (AF), and fluoropolymer. Such materials provide excellent optical transparency, chemical resistance, and mechanical strength, and low refractive index ensuring the device operates effectively under various conditions. Fluoropolymers are particularly advantageous due to their low refractive indices and compatibility with the refractive properties of the light-guiding liquid.

The lightguide assembly can be configured such that the first closure comprises a UV transparent film. Using a UV transparent film can ensure that light emitted from the connected light source can effectively enter the liquid-filled inner lumen without significant loss. A UV-transparent film allows for optimal light propagation, maintaining the device's efficiency in disinfection applications.

Moreover, the lightguide assembly can be configured such that the UV transparent film has a thickness preferably larger than 0.1 mm, more preferably larger than 0.5 mm, and preferably smaller than 5 mm, more preferably smaller than 3 mm, such as 1 mm. Different thicknesses can affect the amount of UV propagation, and may also affect the durability of the film. Depending on the type of application, a specific thickness may be chosen.

In addition, the lightguide assembly can be configured such that the liquid is retained within the closed inner lumen provides illumination externally to the light disinfection device in a 360º radial distribution over at least a part of the length of the elongated tubular body. This uniform radial light emission ensures comprehensive exposure of the surrounding environment or surfaces, making the device highly effective in applications requiring broad-area disinfection or illumination in confined spaces.

The lightguide assembly can be configured such that the connectable light source is a UV light source. A UV light source provides efficient germicidal properties. This makes the device suitable for medical, laboratory, or industrial applications where disinfection is critical.

In an embodiment, the lightguide assembly can be configured such that the connectable light source is a UVC light source. UVC light, which operates within the wavelength range of 200-280 nm, is particularly effective for disinfection as it can disrupt the DNA and RNA of microorganisms, rendering them inactive. Using a UVC light source ensures high efficacy in disinfection tasks, making the device versatile and suitable for demanding disinfection applications.

The present disclosure further relates to a protective cap for protective storage of a liquid filled elongated tubular body, the protective cap comprising an elongated cover for encapsulating the elongated tubular body. The elongated cover comprises a closed distal end, a proximal open end for receiving a distal end of the elongated tubular body, and a proximal connector in connection with the proximal open end configured for engagement with the connector piece of the light disinfection device to form a closed outer lumen encapsulating the elongated tubular body for retaining a protective fluid. As described in the previous sections, one purpose of the protective cap is to avoid air to penetrate into the liquid in the inner lumen of the light disinfection device, thereby preventing the formation of air bubbles which attenuate the light guidance through the device. The protective cap may comprise any one of the features of the protective cap defined herein. The protective cap may be engaged to any tubular body that can benefit from protection during storage or handling.

The present disclosure further relates to a method for preventing the formation of air pockets in a light disinfection device. The method comprising the steps of obtaining a light disinfection device, the light disinfection device comprising an elongated tubular body, said elongated tubular body comprising a liquid, encapsulating the elongated tubular body within a protective cap, and filling the volume between the protective cap and the elongated tubular body with a protective liquid, thereby preventing any liquid from escaping the interface between the elongated tubular body and the protective cap, and preventing air from entering the interface between the elongated tubular body and the protective cap.

The method for preventing the formation of air pockets in a light disinfection device can be adapted, such that the light disinfection device is the light disinfection device defined in any one of the embodiments described herein, and/or such that the protective cap is the protective cap defined in any one of the embodiments described herein.

### Examples

The lightguide assembly can when connected to a light source, specifically UVC, be used for disinfection of medical tubes such as central venous catheters (CVC), peritoneal catheters (PD), parenteral nutrition catheters (PN) and endotracheal tubes (ETT). Venous catheters are before and after use flushed with a transparent 0.9% saline solution. This solution is UV transparent. The elongated tubular part of the assembly can then be inserted into preferable the extension lines of the CVC catheters. When fasten and locked to venous catheter by the Luer connector piece 106 the light source can be switched on and the inner surfaces and lumen of the catheter is swept with UVC light and disinfection takes place. The same procedure can be carried with air pipes such as ETTs. These tubes are both much longer and wider than the extension part of the CVCs and therefore requires a longer exposure time.

### Further details

1. A kit-of-parts in the form of a lightguide assembly comprising
   - a light disinfection device comprising an elongated tubular body and a connector piece connectable to a light source, the elongated tubular body comprising
      ∘ a transparent tube wall having a refractive index n₂,
      ∘ a proximal end closed by a first closure and connectable to the connector piece,
      ∘ a distal end closed by a second closure, wherein the tube wall, the first closure and the second closure define a closed inner lumen, and
      ∘ a light guiding liquid retained within the closed inner lumen, the light guiding liquid having a refractive index n₁, wherein n₂/n₁ < 1 so that the elongated tubular body is configured for guiding light from the light source inside the liquid filled inner lumen along a longitudinal direction of the elongated tubular body, wherein a part of the light is emitted through the tube wall, such that the light disinfection device is configured for emitting light radially from and along the length of the elongated tubular body,
   - a protective cap for safe storage of the elongated tubular body, the protective cap comprising an elongated cover for encapsulating the elongated tubular body, the elongated cover comprising,
      ∘ a closed distal end,
      ∘ a proximal open end for receiving the distal end of the elongated tubular body, and
      ∘ a proximal connector in connection with the proximal open end configured for engagement with the connector piece of the light disinfection device to form a closed outer lumen encapsulating the elongated tubular body for retaining a protective fluid.
2. The kit-of-parts according to item 1, wherein the protective fluid is a protective liquid.
3. The kit-of-parts according to any one of the preceding items, wherein the protective cap is configured to seal the elongated body, thereby preventing any liquid from escaping the closed outer lumen encapsulating the elongated tubular body, and preventing air from entering the closed inner lumen and the closed outer lumen.
4. The kit-of-parts according to any one of the preceding items, further comprising a protective sleeve configured to encapsulate the light disinfection device.
5. The kit-of-parts according to item 4, wherein the protective sleeve is configured to be selectively elongated or contracted along the axis of the light disinfection device.
6. The kit-of-parts according to any one of the preceding items, wherein the protective fluid is the same as the light guiding liquid.
7. The kit-of-parts according to any one of the preceding items, wherein the protective fluid and/or the light guiding liquid is an ionic solution, such as a saline solution.
8. The kit-of-parts according to any one of the preceding items, wherein the protective fluid and/or the light guiding liquid is a saline solution, wherein a salt concentration of the saline solution is at least 10 g/100 ml, or at least 20 g/100 ml, or at least 30 g/100 ml.
9. The kit-of-parts according to any one of the preceding items, wherein the protective cap is made of a polymer.
10. The kit-of-parts according to any one of the preceding items, wherein the protective cap is made of a material selected from the group of: polypropylene, polystyrene, nylon, polycarbonate and methacrylate.
11. The kit-of-parts according to any one of the preceding items, wherein the protective cap is made of a transparent material.
12. The kit-of-parts according to any one of the preceding items, wherein the connector piece of the light disinfection device is a Luer lock-type connector.
13. The kit-of-parts according to any one of the preceding items, wherein the proximal connector of the protective cap is a Luer lock-type connector.
14. The kit-of-parts according to any one of the preceding items, wherein the connector piece is configured for connecting the light disinfection device to a medical device or to a medical tube, such as a catheter.
15. The kit-of-parts according to item 14, wherein the connector piece is configured for secure connection with a venous catheter having a lumen.
16. The kit-of-parts according to any one of the preceding items, wherein the connector piece comprises a threaded or snap-fit locking mechanism for securing the light disinfection device to the light source.
17. The kit-of-parts according to any one of the preceding items, wherein the connector piece of the light disinfection device comprises a threaded or snap-fit locking mechanism for engagement with the proximal connector of the protective cap.
18. The kit-of-parts according to any one of the preceding items, wherein the connector piece of the light disinfection device is configured for sliding along the elongated tubular body, between the first closure and the second closure.
19. The kit-of-parts according to any one of the preceding items, wherein the connector piece of the light disinfection device comprises a male connector part for engaging with a female connector part of the light source.
20. The kit-of-parts according to any one of the preceding items, wherein the proximal connector of the protective cap comprises a male connector part for engaging with a female connector part of the connector piece of the light disinfection device.
21. The kit-of-parts according to any one of the preceding items, wherein the connector piece of the light disinfection device and/or the proximal connector of the protective cap is made of a UV-resistant material.
22. The kit-of-parts according to any one of the preceding items, wherein the connector piece of the light disinfection device comprises an adjustable locking collar for enabling varying degrees of tightness when securing the proximal end of the elongated tubular body.
23. The kit-of-parts according to any one of the preceding items, wherein the connector piece of the light disinfection device comprises multiple connection interfaces, such as bayonet and threaded connections for accommodating different types of light sources.
24. The kit-of-parts according to any one of the preceding items, wherein the connector piece of the light disinfection device comprises a rotatable joint, enabling the light disinfection device to rotate 360º while maintaining a secure connection to the light source.
25. The kit-of-parts according to any one of the preceding items, wherein the connector piece comprises a controllable shutter configured to block transmission of light to first closure of the light disinfection device.
26. The kit-of-parts according to any one of the preceding items, wherein the light disinfection device comprising a light coupling element, the light coupling element comprising
   - an optical window,
   - a distal end configured for engagement with the first closure, and
   - a proximal end configured for engagement with the light source, such that the light from the light source is guided into the light disinfection device.
27. The kit-of-parts according to item 26, wherein the distal end of the light coupling element comprises a seal.
28. The kit-of-parts according to any one of the items 25-27, wherein the light coupling element comprises an O-ring positioned between the optical window and the distal end of the light guiding element.
29. The kit-of-parts according to any one of the items 25-28, wherein the connector piece is configured to secure the light coupling element between the light source and the elongated tubular body.
30. The kit-of-parts according to any one of the preceding items, wherein the material of the elongated tubular body is selected from the group of: Teflon, Fluorinated ethylene propylene (FEP), polytetrafluoroethylene (PTFE), amorphous fluoropolymers (AF), and fluoropolymer.
31. The kit-of-parts according to any one of the preceding items, wherein the first closure is a UV transparent film.
32. The kit-of-parts according to item 31, wherein the UV transparent film has a thickness preferably larger than 0.1 mm, more preferably larger than 0.5 mm, and preferably smaller than 5 mm, more preferably smaller than 3 mm, such as 1 mm.
33. The kit-of-parts according to any one of the preceding items, wherein the liquid retained within the closed inner lumen provides illumination external to the light disinfection device in a 360º radial distribution over at least a part of the length of the elongated tubular body.
34. The kit-of-parts according to any one of the preceding items, wherein the connectable light source is a UV light source.
35. The kit-of-parts according to any one of the preceding items, wherein the connectable light source is a UVC light source.
36. A protective cap for protective storage of a liquid filled elongated tubular body, the protective cap comprising an elongated cover for encapsulating the elongated tubular body, the elongated cover comprising,
   - a closed distal end,
   - a proximal open end for receiving a distal end of the elongated tubular body, and
   - a proximal connector in connection with the proximal open end configured for engagement with the connector piece of the light disinfection device to form a closed outer lumen encapsulating the elongated tubular body for retaining a protective fluid
37. The protective cap according to item 36, comprising any one of the features of the protective cap defined in any one of the items 1-35.
38. A method for preventing the formation of air pockets in a light disinfection device, the method comprising the steps
   - obtaining a light disinfection device, the light disinfection device comprising an elongated tubular body, said elongated tubular body comprising a liquid,
   - encapsulating the elongated tubular body within a protective cap, and
   - filling the interface between the protective cap and the elongated tubular body with a protective liquid, thereby preventing any liquid from escaping the interface between the elongated tubular body and the protective cap, and preventing air from entering the interface between the elongated tubular body and the protective cap.
39. The me method according to item 38, wherein the light disinfection device is the light disinfection device defined in any one of the items 1-35, and/or wherein the protective cap is the protective cap defined in any one of the items 1-35.

## Claims

1. A kit-of-parts in the form of a lightguide assembly comprising
• a light disinfection device comprising an elongated tubular body and a connector piece connectable to a light source, the elongated tubular body comprising
∘ a transparent tube wall having a refractive index n₂,
∘ a proximal end closed by a first closure and connectable to the connector piece,
∘ a distal end closed by a second closure, wherein the tube wall, the first closure and the second closure define a closed inner lumen, and
∘ a light guiding liquid retained within the closed inner lumen, the light guiding liquid having a refractive index n₁, wherein n₂/n₁ < 1 so that the elongated tubular body is configured for guiding light from the light source inside the liquid filled inner lumen along a longitudinal direction of the elongated tubular body, wherein a part of the light is emitted through the tube wall, such that the light disinfection device is configured for emitting light radially from and along the length of the elongated tubular body,
• a protective cap for safe storage of the elongated tubular body, the protective cap comprising an elongated cover for encapsulating the elongated tubular body, the elongated cover comprising,
∘ a closed distal end,
∘ a proximal open end for receiving the distal end of the elongated tubular body, and
∘ a proximal connector in connection with the proximal open end configured for engagement with the connector piece of the light disinfection device to form a closed outer lumen encapsulating the elongated tubular body for retaining a protective fluid.

2. The kit-of-parts according to claim 1, wherein the protective fluid is a protective liquid.

3. The kit-of-parts according to any one of the preceding claims, wherein the protective cap is configured to seal the elongated body, thereby preventing any liquid from escaping the closed outer lumen encapsulating the elongated tubular body, and preventing air from entering the closed inner lumen and the closed outer lumen.

4. The kit-of-parts according to any one of the preceding claims, further comprising a protective sleeve configured to encapsulate the light disinfection device.

5. The kit-of-parts according to claim 4, wherein the protective sleeve is configured to be selectively elongated or contracted along the axis of the light disinfection device.

6. The kit-of-parts according to any one of the preceding claims, wherein the protective fluid is the same as the light guiding liquid.

7. The kit-of-parts according to any one of the preceding claims, wherein the protective fluid and/or the light guiding liquid is an ionic solution, such as a saline solution.

8. The kit-of-parts according to any one of the preceding claims, wherein the protective fluid and/or the light guiding liquid is a saline solution, wherein a salt concentration of the saline solution is at least 10 g/100 ml.

9. The kit-of-parts according to any one of the preceding claims, wherein the protective cap is made of a material selected from the group of: polypropylene, polystyrene, nylon, polycarbonate and methacrylate.

10. The kit-of-parts according to any one of the preceding claims, wherein the protective cap is made of a transparent material.

11. The kit-of-parts according to any one of the preceding claims, wherein the light disinfection device comprising a light coupling element, the light coupling element comprising
• an optical window,
• a distal end configured for engagement with the first closure, and
• a proximal end configured for engagement with the light source, such that the light from the light source is guided into the light disinfection device.

12. A protective cap for protective storage of a liquid filled elongated tubular body, the protective cap comprising an elongated cover for encapsulating the elongated tubular body, the elongated cover comprising,
• a closed distal end,
• a proximal open end for receiving a distal end of the elongated tubular body, and
• a proximal connector in connection with the proximal open end configured for engagement with the connector piece of the light disinfection device to form a closed outer lumen encapsulating the elongated tubular body for retaining a protective fluid

13. The protective cap according to claim 12, comprising any one of the features of the protective cap defined in any one of the claims 1-11.

14. A method for preventing the formation of air pockets in a light disinfection device, the method comprising the steps
• obtaining a light disinfection device, the light disinfection device comprising an elongated tubular body, said elongated tubular body comprising a liquid,
• encapsulating the elongated tubular body within a protective cap, and
• filling the interface between the protective cap and the elongated tubular body with a protective liquid, thereby preventing any liquid from escaping the interface between the elongated tubular body and the protective cap, and preventing air from entering the interface between the elongated tubular body and the protective cap.

15. The method according to claim 14, wherein the light disinfection device is the light disinfection device defined in any one of the claims 1-11, and/or wherein the protective cap is the protective cap defined in any one of the claims 1-11.

## Amended claims

### Amended claims in accordance with Rule 137(2) EPC.

1. **A kit-of-parts** in the form of a lightguide assembly comprising:
- a light disinfection device (100) comprising an elongated tubular body (102) and a connector piece (107) connectable to a light source, the elongated tubular body (102) comprising:
∘ a transparent tube wall (110) having a refractive index n₂,
∘ a proximal end (108) closed by a first closure and connectable to the connector piece (107),
∘ a distal end (109) closed by a second closure, wherein the tube wall (110), the first closure and the second closure define a closed inner lumen, and
∘ a light guiding liquid (111) retained within the closed inner lumen, the light guiding liquid (111) having a refractive index n₁, wherein n₂/n₁ < 1 so that the elongated tubular body (102) is configured for guiding light from the light source inside the liquid filled inner lumen along a longitudinal direction of the elongated tubular body (102), wherein a part of the light is emitted through the tube wall, such that the light disinfection device is configured for emitting light radially from and along the length of the elongated tubular body,
- a protective liquid, and
- a protective cap (204; 400; 500) for safe storage of the elongated tubular body (102), the protective cap comprising an elongated cover (200) for encapsulating the elongated tubular body (102), the elongated cover (200) comprising:
∘ a closed distal end (201),
∘ a proximal open end (202) for receiving the distal end (109) of the elongated tubular body (102), and
∘ a proximal connector (203) in connection with the proximal open end (202) engaged with the connector piece (107) of the light disinfection device (100) to form a closed outer lumen encapsulating the elongated tubular body (102), the closed outer lumen configured for retaining the protective fluid.

2. The kit-of-parts according to any one of the preceding claims, wherein the protective cap (204; 400; 500) is configured to seal the elongated body (102), thereby preventing any liquid from escaping the closed outer lumen encapsulating the elongated tubular body (102), and preventing air from entering the closed inner lumen and the closed outer lumen.

3. The kit-of-parts according to any one of the preceding claims, further comprising a protective sleeve (600; 700; 800) configured to encapsulate the light disinfection device (100).

4. The kit-of-parts according to claim 4, wherein the protective sleeve (600; 700; 800) is configured to be selectively elongated or contracted along the axis of the light disinfection device (100).

5. The kit-of-parts according to any one of the preceding claims, wherein the protective liquid is the same as the light guiding liquid (111).

6. The kit-of-parts according to any one of the preceding claims, wherein the protective liquid and/or the light guiding liquid (111) is an ionic solution, such as a saline solution.

7. The kit-of-parts according to any one of the preceding claims, wherein the protective liquid and/or the light guiding liquid (111) is a saline solution, wherein a salt concentration of the saline solution is at least 10 g/100 ml.

8. The kit-of-parts according to any one of the preceding claims, wherein the protective cap (204; 400; 500) is made of a material selected from the group consisting of polypropylene, polystyrene, nylon, polycarbonate and methacrylate.

9. The kit-of-parts according to any one of the preceding claims, wherein the protective cap (204; 400; 500) is made of a transparent material.

10. The kit-of-parts according to any one of the preceding claims, wherein the light disinfection device (100) comprises a light coupling element, the light coupling element comprising:
- an optical window (103),
- a distal end configured for engagement with the first closure, and
- a proximal end configured for engagement with the light source, such that the light from the light source is guided into the light disinfection device (100).

11. A method for preventing the formation of air pockets in a light disinfection device (100), the method comprising the steps of:
- obtaining a light disinfection device (100) and a protective cap (204; 400; 500) of a kit-of-parts according to any one of the preceding claims, and
- filling a volume between the protective cap (204; 400; 500) and the elongated tubular body (102) of the light disinfection device (100) with the protective liquid, thereby preventing any liquid from escaping the interface between the elongated tubular body (102) and the protective cap (204; 400; 500), and preventing air from entering the interface between the elongated tubular body (102) and the protective cap (204; 400; 500).
